# EUROPEAN PATENT SPECIFICATION

(11) **EP 2 098 864 B1**
(45) Date of publication and mention of the grant of the patent: **28.12.2016**
(21) Application number: 07830409.4
(22) Date of filing: 17.10.2007
(51) Int. Cl.: G01N 33/543, G01N 33/62

(54) **METHOD OF IMMUNOASSAYING URINARY COMPONENT AND REAGENT TO BE USED THEREFOR**
IMMUNTESTVERFAHREN FÜR EINE URINKOMPONENTE UND DAFÜR ZU VERWENDENDES REAGENS
METHODE DE DOSAGE IMMUNOLOGIQUE D'UN COMPOSANT URINAIRE ET REACTIF A UTILISER POUR CETTE METHODE

(30) Priority: 17.10.2006 JP 2006282278
(43) Date of publication of application: 09.09.2009
(73) Proprietor: Tokyo Metropolitan Institute of Medical Science, Tokyo 156-0057 (JP); Tokyo Metropolitan Government, Shinjuku-ku Tokyo 163-8001 (JP)
(72) Inventor: TAKAHASHI, Keiichi, Saitama-shi Saitama 338-0012 (JP); HIRAMATSU, Kyoko, Tokyo 124-0024 (JP); KAWAKITA, Masao, Tokyo 161-0033 (JP); YANAGIYA, Mari, Ibaraki-shi Osaka 567-0806 (JP); KOSAKA, Mieko, Ibaraki-shi Osaka 567-0806 (JP)
(74) Representative: Woods, Geoffrey Corlett
(86) International application number: PCT/JP2007/070675
(87) International publication number: WO 2008/047941

(56) References cited:
- EP-A2- 0 038 181
- WO-A1-01/44815
- GB-A- 2 042 170
- JP-A- 55 160 853
- JP-A- 2006 038 594
- KYOKO HIRAMATSU: "Development of a Sensitive and Accurate Enzyme-Linked Immunosorbent Assay (ELISA) System That Can Replace HPLC Analysis for the Determination of N1, N12-Diacetylspermine in Human Urine" JOURNAL OF BIOCHEMISTRY, JAPANESE BIOCHEMICAL SOCIETY / OUP, TOKYO; JP, vol. 124, no. 1, 1 January 1998 (1998-01-01), pages 231-236, XP002979923 ISSN: 0021-924X
- BOWYER R C ET AL: "Factors affecting the solubility of ammonium acid urate", CLINICA CHIMICA ACTA, ELSEVIER BV, AMSTERDAM, NL, vol. 95, no. 1, 2 July 1979 (1979-07-02), pages 17-22, XP024785962, ISSN: 0009-8981, DOI: 10.1016/0009-8981(79)90331-0 [retrieved on 1979-07-02]
- MAKINO K ET AL: "ESR AND SPIN TRAPPING STUDY OF GAMMA IRRADIATED AQUEOUS HYDANTOIN SOLUTIONS KETO ENOL EQUILIBRIUM AND POST RADIOLYSIS GROWTH", RADIATION RESEARCH, ACADEMIC PRESS INC, US, vol. 95, no. 3, 1 September 1983 (1983-09-01), pages 519-529, XP008175828, ISSN: 0033-7587, DOI: 10.2307/3576097
- OKAYAMA K-I ET AL: "Sourness of foods and its electrochemical measurement. VI. Application of organic weak bases (imidazole and 2-aminopyridine) in an aqueous solution system for electrochemical measurement of organic acid content. (translated)", NIPPON SHOKUHIN KOGYO GAKKAISHI - JOURNAL OF FOOD SCIENCE AND TECHNOLOGY, NIPPON SHOKUHIN KAGAKUKOUGAKUKAI, JP, vol. 27, no. 4, 1 January 1980 (1980-01-01), pages 179-182, XP008175886, ISSN: 0029-0394
- SHUKLA P C ET AL: "Electrokinetic studies of aqueous hippuric acid solutions across urinary bladder membranes", MODELLING, MEASUREMENT AND CONTROL, C, AMSE PRESS, FRANCE, vol. 19, no. 2, 1 January 1990 (1990-01-01), pages 39-55, XP008175829, ISSN: 0761-2524

## Description

### TECHNICAL FIELD

The present invention relates to a method for immunological measurement of urine components and the use of a reagent for use therein.

### BACKGROUND ART

In fields such as laboratory testing, immunological measurement is used as one method of measuring the quantity of substances. There are many immunological measurement methods, such as the RIA method, the EIA method, the immunoturbidimetric method, the latex agglutination method and the colloidal metal immunoassay. These measurement methods use an antigen/antibody reaction between an antigen (or antibody) which is the substance to be measured in a biological sample and its corresponding antibody (or antigen). In various tests in the field of laboratory testing, automation and the reduction of measurement time are being pursued. Of the various tests, the latex agglutination method and the colloidal metal immunoassay are suited to automation because they do not involve the separation or washing of reaction solutions. In these immunological measurements, the concentration of a substance to be measured (hereinafter, sometimes called "target substance") is determined by comparing the calibration curve that represents the relationship between the values obtained by measuring a standard solution (e.g. absorbances) and concentrations thereof with the measured value of the target substance in the sample. When a target substance in a biological sample has given or is expected to give a value that exceeds the concentration range of the calibration curve, the biological sample must be diluted with a diluting solution until it reaches a concentration within the range of the predetermined calibration curve. In these measurements, pre-prepared control solutions are sometimes measured in order to confirm the accuracy of measurements. In general, a normal saline or a buffer solution is used as a solvent for this type of standard preparations such as standard samples, diluting solution and control solution.

However, there exists a problem due to the fact that a normal saline or a buffer solution has different properties in terms of ingredients, viscosity, specific gravity and so forth from a biological specimen to be measured, which leads to a difference between the reactivity with reagents of the biological specimen and the reactivity with reagents of the standard preparation, diluted sample or control. Measurements cannot then be performed accurately, leading to deviation from the true values. It is therefore necessary to prepare a standard preparation (solution), a diluted solution, or a control having reactivity with reagents identical to the reactivity of biological sample.

Detection and measurement of a target substance in urine as a biological sample are attracting attention because urine is easy to collect. For example, urinary diacetylspermine is one of tumor markers, and immunological methods are used in the measurement thereof (Hiramatsu, K. et al., The Journal of Biochemistry (J. Biochem.), 124, 231-236 (1998) and Japanese Laid-Open Patent Publication No. 2006-38594). As one reagent used in the measurement of diacetylspermine, a "Urinary Diacetylspermidine ELISA Kit" is available commercially from Trans Genic Inc. (sold by Funakoshi Corporation).

In the immunological measurement of a target substance in urine as a biological sample, difference in immunoreactivity also occurs when a normal saline or a buffer solution is used in sample preparations, diluting solution, etc. This makes accurate measurement impossible, leading to deviation from the true value.

GB 2 042 170 discloses a reagent for tests utilizing latex agglutination.

Hiramatsu et al. (1998) J. Biochem 124:231-236 discloses an enzyme-linked immunosorbent assay (ELISA) system for the determination of diacetylspermine in human urine.

EP 0 038 181 discloses an immunoassay involving agglutination of finely divided particles.

WO 01/44815 discloses an immunoassay method for detection of *Helicobacter pylori* antigens in serum samples.

Bowyer et al. (1979) Clinica Chimica Acta, 95:17-22 discusses factors affecting the solubility of ammonium acid urate.

Makino et al. (1983) Radiation Research 95:519-529 describes an ESR and spin trapping study of y-irradiated aqueous hydantoin solutions.

Okayama et al. (1980) Nippon Shokuhin Kogyo Gakkaishi 27: 179-182 discloses the application of organic weak base aqueous solution system for electrochemical measurement of organic acid content.

Shukla et al. (1990) Modelling, Simulation & Control, 19: 39-55 describes electrokinetic studies of aqueous hippuric acid solutions across urinary bladder membranes.

### DISCLOSURE OF THE INVENTION

The purpose of the present invention is to provide a method for accurately measuring the quantity of a target substance in a urine sample or diluted urine sample in the immunological measurement of urine components, as well as a reagent for use therein.

That is to say, the present invention is as follows.

The present invention provides the use of a reagent in the immunological measurement of a urine component, wherein said urine component is diacetylspermine, said immunological measurement is carried out by a colloidal gold agglutination method and said reagent comprises at least one substance selected from urea, potassium thiocyanate, guanidinium hydrochloride, imidazole, hydantoin, hippuric acid and sodium urate.

In one embodiment, the protein concentration of the above-mentioned reagent is 0.01 to 1 w/v %. In still another embodiment, the above-mentioned protein is bovine serum albumin.

In one embodiment, the salt concentration of the above-mentioned reagent is 0.01 to 10 w/v %.

In an embodiment, the above-mentioned reagent is a standard solution, a diluting solution or a reaction reagent.

Further, the present invention provides a method for immunologically measuring the quantity of a urine component, comprising the following steps:
(a) adding a reaction reagent to a urine sample or a mixed solution of a urine sample and a diluting solution to generate a reaction solution and then measuring either the change caused by immunological reaction or the reaction product;
(b) adding the reaction reagent to a standard solution or a mixed solution of a standard solution and a diluting solution to generate a reaction solution and then measuring either the change caused by immunological reaction or the reaction product; and
(c) comparing the measurement results of (a) to the measurement results of (b) to determine the quantity of the urine component in the urine sample,
wherein at least one reagent selected from the group consisting of the diluting solution, the standard solution and the reaction reagent comprises urea, and urea is contained in the reaction solution generated in (b), and wherein said urine component is diacetylspermine and said immunological measurement is carried out by a colloidal gold agglutination method.

In one embodiment, the protein concentration of the reaction solution generated in (b) above is 0.00025 to 0.03 w/v %. In still another embodiment, the above-mentioned protein is bovine serum albumin.

In another embodiment, the salt concentration of the reaction solution generated in (b) above is 0.00025 to 0.3 w/v %.

In one embodiment, the above-mentioned urine is human urine.

In one embodiment, the above-mentioned diluting solution, standard solution and/or reaction reagent further comprises at least one substance selected from potassium thiocyanate, guanidinium hydrochloride, imidazole, hydantoin, hippuric acid and sodium urate.

### BRIEF DESCRIPTION OF THE DRAWINGS

Fig. 1 is a graph showing measured values of urinary diacetylspermine concentration when a diluting solution or standard solution contains added urea at various concentrations.
Fig. 2 is a graph showing the relationship between urinary diacetylsperimine concentration and the absorbance that was measured using a set of standard solutions prepared with a diluting solution containing urea.
Fig. 3 is a graph showing measured values of urinary diacetylspermine concentration obtained using standard solutions prepared with diluting solutions containing various added substances.

### BEST MODES FOR CARRYING OUT THE INVENTION

Hereinbelow, the present invention will be described in detail. The following descriptions are not intended to limit the scope of the present invention. Other than the following examples, the present invention may be appropriately modified and carried out within a range within the scope of the claims.

Throughout the present specification, the percentage (%) noted to show the content of a substance in a solution is, unless otherwise specified, w/v %.

### 1. A Reagent for Immunological Measurement of Urine Components

Specific examples of the reagent used in the present invention include a standard solution, a diluting solution and a reaction reagent.

The term "standard solution" means a solution for use in the production of a calibration curve (hereinafter, also called "standard curve") for a target substance to be measured in immunological measurement of urinary substances (hereinafter, also called "urine components"). A standard solution contains the target substance at a specific concentration. A standard solution may be a solution containing the target substance at a single concentration or a set of solutions containing the target substance at several concentrations.

The term "diluting solution" means a solution used to dilute a urine sample or a standard solution so that the concentration of the target substance in the urine sample falls within the concentration range of the calibration curve. Diluting solutions are usually used to dilute samples. They may also be used to perform dissolution of the target substance to thereby prepare a standard solution, or to dilute a standard solution to thereby prepare solutions containing various concentrations of the target substance.

The reaction reagent may be a reagent solution containing an immunological partner that corresponds to the target substance to be measured. In the competition method, the reaction reagent may be a reagent solution containing an immunological partner that corresponds to the target substance and a reagent solution containing a substance that competes with the target substance for the above-mentioned immunological partner.

According to the present invention, for immunological measurement of urine components, at least one of the following substances may be contained in the reaction solution to induce immunological reaction of urine components: urea, potassium thiocyanate, guanidinium hydrochloride, imidazole, hydantoin, hippuric acid and sodium urate. Therefore, it is sufficient that any one of the diluting solution, standard solution and reaction reagent used in the immunological measurement of urine components contains the above-mentioned substance.

Hereinbelow, the reagents used in the present invention are individually explained with respect to the diluting solution, standard solution and reaction reagent. However, this does not mean that all the diluting solution, standard solution and reaction reagent used in the immunological measurement of urine components must contain the above-mentioned substance.

The standard solution or diluting solution of the present invention comprises at least one substance selected from urea, potassium thiocyanate, guanidinium hydrochloride, imidazole, hydantoin, hippuric acid and sodium urate. These substances may be contained alone or in combination. These substances are contained at a concentration that eliminates or almost eliminates differences in the reactivity of the components in immunoreaction in the standard solution or diluting solution compared to that when a urine sample is used. A concentration is preferable that allows the rate of deviation between the measured value and true value of the target substance to be within ±15%. More preferable is within ±10%, still more preferable is within ±8%. For example, when the above-mentioned substances are contained alone, the concentrations may be the following: urea at 0.2 to 12%, more preferably 0.5 to 10%; potassium thiocyanate at 0.2 to 12%; more preferably 0.5 to 10%; guanidinium hydrochloride at 0.2 to 12%, more preferably 0.5 to 10%; imidazole at 0.2 to 12%, more preferably 0.5 to 10%; hydantoin at 0.2 to 12%, more preferably 0.5 to 10%; hippuric acid at 0.001 to 0.2%, more preferably 0.005 to 0.1%; and sodium urate at 0.001 to 0.2%, more preferably 0.005 to 0.1%. When these substances are used in combination, the concentration may be as low as 1/10 of each of the above-mentioned concentrations.

These solutions may also contain proteins. The preferred concentration thereof is 0.01 to 1%, more preferably 0.05 to 0.5%. Examples of proteins include albumin, casein, globulin, hemocyanin, and thyroglobulin. Of these proteins, albumin is preferred; especially preferred is bovine serum albumin (BSA).

These solutions may also contain salts. The preferred concentration thereof is 0.01 to 10%, more preferably 0.05 to 5%. Examples of salts include inorganic salts such as sodium chloride and potassium chloride.

To maintain the pH, these solutions may contain a buffer. Examples of buffer that can be preferably used include phosphate buffer, Tris-HCl buffer, succinate buffer, or Good's buffer such as glycylglycine, MES (2-(N-morpholino)ethane sulfonic acid), HEPES (N-2-hydroxyethyl-piperazine-N'-ethane sulfonic acid), TES (N-tris(hydroxymethyl)methyl-2-aminoethanesulfonic acid), MOPS (3-(N-morpholino)propanesulfonic acid) and PIPES (piperazine-1,4-bis(2-ethane sulfonic acid)). The pH thereof is preferably 3.0 to 7.0. The buffer is used preferably at a final concentration of 5 to 500 mM

Other substances may also be contained, including sodium azide as an antiseptic agent, EDTA as a chelating agent, or organic acids, sugars or amino acids.

The standard solution is used for comparison with samples and may therefore contain the target substance to be measured at preferred concentrations. The standard solution can also be prepared by adding the target substance to the diluting solution to give a preferred concentration. For example, when the target substance is diacetylspermine, the standard solution will contain diacetylspermine at a concentration preferred for measurement. The preferred concentration is 0 to 1000 nM. In general, in order to prepare the calibration curve, a set of standard solutions containing the target substance at different concentrations are necessary. The concentrations can be appropriately selected by one skilled in the art, and the standard solutions can be prepared from a standard solution of a higher concentration by appropriately diluting it with the diluting solution.

The above-mentioned standard solutions and diluting solutions may also be used as control solutions in order to verify the accuracy of measurements.

The reaction agent used in the present invention comprises at least one substance selected from urea, potassium thiocyanate, guanidinium hydrochloride, imidazole, hydantoin, hippuric acid, and sodium urate. These substances can be contained alone or in combination. Irrespective of whether a diluting solution is or is not added to either a sample or a standard solution, these substances are contained at a concentration that gives little or no difference in the respective reactivity of the components in the sample or standard solution in the immunoreaction when a reaction agent is added to the sample or the standard solution for reaction. A concentration that allows the rate of deviation between the measured value and true value of the target substance to be within ±15% is preferable. More preferable is within ±10%, still more preferable is within ±8%. If two or more reaction reagents are needed to measure the target substance, any one or all of the reaction reagents (i.e., at least one of the reaction reagents) may include the above-mentioned substances.

When the above-mentioned substances are used alone, the reaction reagent can contain, for example, urea at 0.1 to 3%, more preferably 0.5 to 2%; potassium thiocyanate at 0.1 to 3%; more preferably 0.5 to 2%; guanidinium hydrochloride at 0.1 to 3%, more preferably 0.5 to 2%; imidazole at 0.1 to 3%, more preferably 0.5 to 2%; hydantoin at 0.1 to 3%, more preferably 0.5 to 2%; hippuric acid at 0.0005 to 0.2%, more preferably 0.0007 to 0.1%; and sodium urate at 0.0005 to 0.2%, more preferably 0.0007 to 0.1%. When these substances are used in combination, the concentration may be as low as 1/10 of each of the above-mentioned concentrations.

Except for including the above substances, the reaction reagent may, if necessary, have a known composition for the measurement of a target substance to be measured. For example, an antiseptic agent, a chelating agent, an organic acid, a sugar, or an amino acid may be included therein in the same manner as in the above-mentioned standard solution or diluting solution.

### 2. Urine Component Measurement Kit

The above-described diluting solution, standard solution and reaction reagent may be included in a kit for use in immunological measurement of urine components, which comprises at least one of those three.

The standard solution included in the kit may be one solution containing the target substance to be measured at the preferred maximum concentration. This may be serially diluted and used for preparing standard solutions with lower concentrations. Alternatively, the kit may comprise, as standard solutions, several solutions containing the target substance at several concentrations for use in preparation of the calibration curve.

The kit may comprise, in lieu of the standard solution containing the above-mentioned substance, both a preparation of the target substance and a diluting solution containing the above-mentioned substance to be used for preparing a standard solution. As a matter of course, the kit may comprise the diluting solution containing the above-mentioned substance together with the standard solution containing the target substance at a specific concentration, and the diluting solution may be used to dilute the standard solution and samples.

The kit may include the reaction reagent containing the above-mentioned substance alone and may be used together with a conventional standard solution and/or dilution solution. Alternatively, the kit may include the reaction reagent together with the standard solution containing the above-mentioned substance and/or the diluting solution containing the above-mentioned substance.

In addition to reagents containing the above-mentioned substances (e.g., the diluting solution, standard solution, or reaction reagent), the kit may also include other components (reagents or devices) necessary for immunological measurement of the target substance to be measured. An instruction manual showing measurement procedures may also be included.

### 3. The Immunological Measurement Method for Urine Components

Immunological measurement of urine components makes use of the immunoreaction that occurs between a substance to be measured (for example, an antigen or antibody) and its corresponding immunological partner (for example an antibody that corresponds to the above-mentioned antigen, or an antigen that corresponds to the above-mentioned antibody). There are two methods of immunological measurement: direct measurement wherein a substance to be measured is detected directly through detection of the immunoreaction between the substance and its corresponding immunological partner; and indirect measurement wherein a substance to be measured is indirectly detected by allowing the substance to coexist with its competitive substance and detecting the competitive immunoreaction. In this measurement, a urine sample is combined with a reaction reagent containing the immunological partner that corresponds to the substance to be measured (in the competition method, a reaction reagent containing a substance with which the substance to be measured is competing for its immunological partner is also included) to prepare a reaction solution. Then, the change induced by immunological reaction or the quantity of the reaction product in the reaction solution is measured. Thus, the quantity of the target substance contained in the urine sample is determined. An optical means is the preferable means of measurement.

The quantity (normally in terms of concentration) of the target substance can be determined by measuring immunological reaction-induced change or quantity of product in the reaction solution and compareing the measured value obtained when the urine sample was used with the measured value using a standard preparation (normally in solution) instead of a urine sample to generate a reaction solution. For example, the quantity of the target substance in the urine sample can be determined from the measured value (e.g. absorbance) of the target substance in the urine sample in reference to the calibration curve. The calibration curve that shows the relationship between the measured values and the quantity of the target substance, is prepared by using at least two concentrations of standard preparation instead of a urine sample, and measuring immunological reaction-induced change or product quantity in the reaction solution. When the target substance in a urine sample has given or is expected to give a value that exceeds the concentration range of the calibration curve, the urine sample may be diluted with a diluting solution until it reaches a concentration within the range of the predetermined calibration curve.

In the method of the present invention, to ensure that deviation does not occur between measured values and true values, in particular with regard to the standard preparation, it is sufficient that a reaction solution used to cause an immunological reaction of a urine component comprises at least one of the following substances: urea, potassium thiocyanate, guanidinium hydrochloride, imidazole, hydantoin, hippuric acid and sodium urate. Accordingly, at least one of the diluting solution, the standard solution, and the reaction reagent contains the above-mentioned substance. Ultimately, the above-mentioned reaction solution may include, for example, if alone, urea at 0.0056 to 0.34%, more preferably 0.014 to 0.28%; potassium thiocyanate at 0.0056 to 0.34%; more preferably 0.014 to 0.28%; guanidinium hydrochloride at 0.0056 to 0.34%, more preferably 0.014 to 0.28%; imidazole at 0.0056 to 0.34%, more preferably 0.014 to 0.28%; hydantoin at 0.0056 to 0.34%, more preferably 0.014 to 0.28%; hippuric acid at 0.000028 to 0.0056%, more preferably 0.00014 to 0.0028%; and sodium urate at 0.000028 to 0.0056%, more preferably 0.00014 to 0.0028%. When these substances are used in combination, a concentration as low as 1/10 of each of the above-mentioned concentrations may be used.

Proteins may also be included in the above-mentioned reaction solution. The preferred concentration thereof is 0.00025 to 0.03%, more preferably 0.001 to 0.01%. Examples of proteins include albumin, casein, globulin, hemocyanin, and thyroglobulin. Of these proteins, albumin is preferred, especially preferred is bovine serum albumin (BSA).

The above-mentioned reaction solution may also include salts. The preferred concentration thereof is 0.00025 to 0.3%, more preferably 0.001 to 0.1%. Examples of salts include inorganic salts such as sodium chloride and potassium chloride.

When a reaction reagent containing the above-mentioned substances is used as a reagent for measuring urine components, purified water, normal saline or a buffer solution may be used to dilute the urine sample.

Although urine of various animals may be used as samples for measurement in the method of the present invention, human urine is preferable.

In the present invention, a urine component to be immunologically measured is not particularly limited, as long as it can be present in urine and can be immunologically measured. Even polymers such as proteins, lipids and polysaccharides can be measured. Immunologically measurable substances include, for example, various antigens, antibodies, receptors, and enzymes. An example is diacetylspermine.

Examples of immunological measurement methods applicable to the present invention include the RIA method, the EIA method, the turbidimetric immunoassay, the latex agglutination method, and the colloidal metal agglutination method. Examples of colloidal metals used in the colloidal metal agglutination method include colloids of gold, silver, selenium and the like. Colloidal gold is desirable from the point of ease of use. Immunoagglutination methods which have no reaction solution separation step or washing step are preferable because automation of testing is easily achieved; especially preferable are the latex agglutination method and the colloidal gold agglutination method. In immunoagglutination methods in which no reaction solution separation or washing operation is performed, solution components such as the sample and the standard solution are retained in the reaction solution until the end of measurement. Accordingly, the use of the reagent of the present invention for immunoagglutination method is more preferable.

For example, when measuring a target substance which is an antigen (or antibody) by the latex agglutination method or colloidal metal agglutination method, the antibody (or antigen) corresponding to the antigen (or antibody) to be measured is bound in advance with the latex or colloidal metal serving as a carrier. For example, in the case of the colloidal gold agglutination method, labeled antibodies (or labeled antigens) bound to colloidal gold in advance are agglutinated via the antigen (or antibody) which is the target substance to be measured. Color differences (tone changes) that occur at this point are optically measured, and quantities of the antigen and antibody are determined.

When diacetylspermine is immunologically measured, use of the method disclosed in Japanese Laid-Open Patent Publication No. 2006-38594 is preferable.

Hereinbelow, the present invention will be more specifically described with reference to the following examples. However, these examples are not intended to limit the scope of the present invention.

First, the immunological measurements used in the present examples are explained in detail below (Reference Examples 1 to 6).

### [Reference Example 1]

### <Preparation of Acetylspermine-bound BSA>

Acetylspermine-bound BSA was prepared in accordance with the method disclosed in "Hiramatsu, K., et al., The Journal of Biochemistry (J. Biochem.), 1998, 124, pp. 231 to 236. First, 200 mg of the carrier protein BSA was mixed with 15 mg of S-acetylmercaptosuccinic anhydride in 0.1 M phosphate buffer (pH 7.0) and stirred for 30 minutes at room temperature to produce S-mercaptosuccinic acid (AMS)-BSA complex. In a separate procedure, 45 mg of a bifunctional crosslinking reagent GMBS was mixed with 147 mg of N-acetylsperimine trihydrochloride in tetrahydrofuran/50 mM phosphate buffer (pH 7.0) (1:1 (v/v)) for 30 minutes at room temperature to produce an acetylspermine-GMB conjugate. Then, 4.2 mg of hydroxylamine hydrochloride was added to 200 mg of AMS-BSA to yield mercaptosuccinyl-BSA (MS-BSA). Subsequently, acetylspermine-GMB conjugate was mixed with MS-BSA, and the immunizing antigen acetylspermine-GMB-BSA, in which N-acetylspermine was conjugated via acylamide-linkage, was produced. Thus, an immunizimg antigen having multiple side chains with structure analogous to diacetylspermine was produced by binding acetylspermine to the carrier through acylamide-linkage.

The concentration of acetylspermine-bound BSA was calculated based on an A₂₈₀ value wherein A₂₈₀, which is the absorbance (light path length 1 cm) at 280 nm, of 1 mg/mL BSA solution was taken as 0.66.

### [Reference Example 2]

### <Preparation of Anti-Diacetylspermine Monoclonal Antibodies>

An emulsified mixture of the acetylspermine-bound BSA obtained in Reference Example 1 above and an adjuvant was administered to mice. The administration was carried out four times at intervals of two weeks. Antibody-producing cells were collected four days after the final immunization.

Subsequently, the collected antibody-producing cells were fused with myeloma cells to obtain a hybridoma. Regarding cell fusion, 1×10⁶ to 1×10 cells/mL antibody-producing cells and 2×10⁵ to 2×10⁶ cells/mL myeloma cells were mixed in an animal cell culture medium (RPMI-1640 culture medium) not containing serum, and cell fusion was initiated by addition of a cell fusion promoter (polyethyleneglycol of 1000 to 6000 Da). After cell fusion, the hybridoma producing anti-diacetylspermine antibody was selected from the cells as described below: the cell suspension was diluted with RPMI-1640 culture medium containing fetal bovine serum and inoculated on a microtiter plate. HAT selection medium was added to each well and cultivation was performed. After approximately two weeks, the cells grown therein were obtained as hybridoma. A portion of the culture supernatant of the multiplied hybrioma culture was collected and screened for antibodies that react with diacetylspermine by enzyme immunoassay. Fused cells were cloned through limiting dilution. A hybridoma that exhibited strong reactivity with diacetylspermine and (1) whose cross-reaction with N¹-acetylspermine is 0.1% or below, and/or (2) the sum of interference with measurement results caused by cross-reaction with urinary diacetylspermine analogue is 5% or below, was selected and established. Monoclonal antibody was recovered from the established hybridoma after cultivation at 37°C with 5%CO₂ for 7 to 14 days. The collected antibody was purified through ammonium sulfate fractionation.

### [Reference Example 3]

### <Preparation of Colloidal Gold Fluid>

2 mL of 10% chloroauric acid solution was added to 1 L of distilled water at 95°C while stirring. 1 minute later, 10 mL of 2% sodium citrate solution was added, and, after stirring for another 20 minutes, the reaction mixture was chilled to 30°C. After being chilled, the pH of the reaction mixture was adjusted to 7.1 with 0.1 M potassium carbonate solution.

### [Reference Example 4]

### <Preparation of Reagent No. 1>

250 mM MES (pH 6.2) (containing 0.2% EDTA, 0.2% TWEEN80, 0.001% DTT, 0.025% 1-thioglycerol and 0.01% sodium azide) containing 66.0 ng/mL acetylspermine-bound BSA prepared in Reference Example 1 above, 2.0% sodium chloride and 1.85% polyethyleneglycol 20000 (PEG) (PEG concentration varied slightly by lot in order to obtain desired measurement sensitivity) was prepared to yield Reagent No. 1.

### [Reference Example 5]

### <Preparation of Reagent No. 2>

The anti-diacetylspermine antibody prepared in Reference Example 2 above was diluted with 10 mM HEPES (pH 7.1) (containing 0.05% sodium azide) to give a concentration of 40 µg/mL. 100mL of this fluid was added to 1 L of the colloidal gold fluid prepared in Reference Example 3 above. The mixture was put in cold storage for two hours with stirring. 110 mL of 10 mM HEPES (pH 7.1) containing 5.46% mannitol, 0.5% BSA and 0.05% sodium azide was added to this mixture. The resultant mixture was then stirred at 37°C for 90 minutes, centrifuged at 8000 rpm for 40 minutes and the supernatant was then removed therefrom. Approximately 1 L of 5 mM HEPES (pH 7.5) containing 3% mannitol, 0.1% BSA and 0.05% sodium azide (solution A) was added to the obtained residue. After the antibody-bound colloidal gold was dispersed, solution A was centrifuged at 8000 rpm for 40 minutes and the supernatant was then removed therefrom. The residue was dispersed in solution A; solution A was added to the anti diacetylspermine antibody-bound colloidal gold dispersion liquid so that absorbance at 540 nm would be 1.0 when diluted 30-fold with purified water, thereby yielding an antibody-bound colloidal gold stock solution.

The antibody-bound colloidal gold stock solution was diluted 3-fold with solution A to produce an anti-diacetylspermine antibody-bound colloidal gold reagent, thereby yielding Reagent No. 2.

### [Reference Example 6]

### <Measurement of Urinary Diacetylspermine>

7 µL of a urine sample containing diacetylspermine and 180 µL of Reagent No. 1 prepared in Reference Example 4 above were mixed together, stirred, and heated to 37°C for approximately 5 minutes. Next, after adding and stirring 60 µL of Reagent No. 2, which was prepared in Reference Example 5 above, the change in optical density at 546 nm as main wavelength and 660 nm as reference wavelength was measured using a Hitachi model 7070 automatic testing unit. Two-point measurement at photometrical points 18 and 31 was carried out, and the difference in absorbance between the two points was determined. Measurements required approximately 10 minutes.

In order to create a standard curve, a diluting solution was used to produce standard solutions. When necessary, diluting solution was used to prepare urine samples containing diacetylspermine.

### [EXAMPLE 1]

### <Effect of Urea Concentration of the Standard Solution and Diluting Solution on the Measurement of Diacetylspermine in Urine Samples>

In this example, the effect of urea concentration of the standard solution and diluting solution on the measurement of diacetylspermine in urine samples was investigated.

### 1-1. Investigations concerning Standard Solutions and Diluting Solutions containing Urea

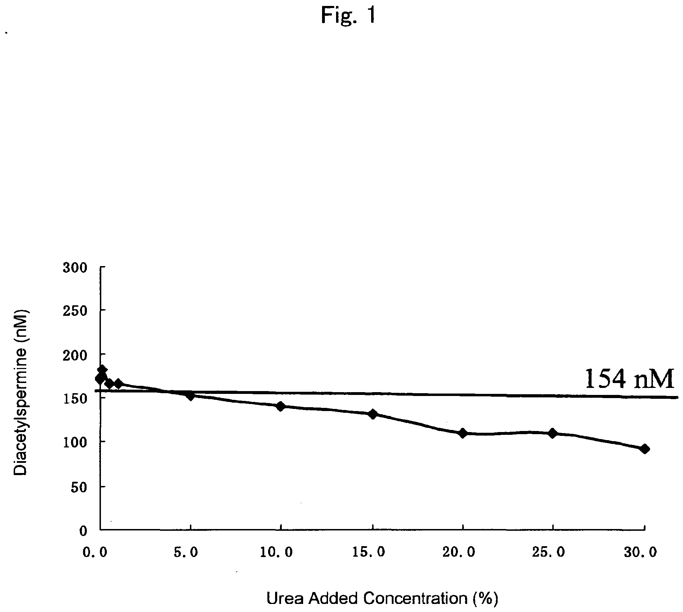

Urea was added to a solution containing 0.3% BSA, 0.9% sodium chloride and 0.2 M MES (pH 6.2) to prepare solutions having the following urea concentrations: 0%, 0.01%, 0.05%, 0.1%, 0.5%, 1%, 5%, 10%, 15%, 20%, 25%, and 30%. Each urea solution thus obtained was used as a diluting solution. Using diluting solutions with various urea concentrations, standard solutions containing diacetylspermine at the following concentrations were prepared: 0 nM, 10 nM, 50 nM, 200 nM, 400 nM, and 1000 nM A control urine sample containing 154 nM diacetylspermine as determined with the above-mentioned Reagent No. 1 (Reference Example 4) and Reagent No. 2 (Reference Example 5) was used to evaluate the effect of urea. The control urine sample consists of pooled urine from healthy volunteers, and the diacetylspermine concentration of which was evaluated and verified through the use of a calibration curve created using a standard preparation quantified by mass spectrometry. For each of the above-mentioned urea concentrations, 6 standard solutions and urine samples were measured of absorbance differences by adding thereto Reagent No. 1 and Reagent No. 2 in a Hitachi model 7070 automatic testing unit, and the diacetylspermine concentration in each urine sample was calculated using the calibration curve obtained for each of the urea concentrations. The optimum concentration of added urea was determined using the rate of deviation between these measured values and true value (154 nM). These results are shown in Table 1 and Fig. 1.

**Table 1**

| Urea % | Measured Value nM | Deviation % |
|---|---|---|
| 0 | 171.3 | +11.3 |
| 0.01 | 172.7 | +12.2 |
| 0.05 | 174.7 | +13.4 |
| 0.1 | 182.1 | +18.3 |
| 0.5 | 165.5 | +7.4 |
| 1 | 165.4 | +7.4 |
| 5 | 152.4 | -1.0 |
| 10 | 140.9 | -8.5 |
| 15 | 130.8 | -15.1 |
| 20 | 109.8 | -28.7 |
| 25 | 109.8 | -28.7 |
| 30 | 92.5 | -39.9 |
| Standard Value | 154 | |

According to Table 1, at a urea concentration of 0%, the observed concentration of the control urine sample containing 154 nM diacetylspermine was 171.3 nM, and the rate of deviation from the true value was +11.3%. High rates of deviation from the true value were observed as follows: +12.2% at a urea concentration of 0.01%, +13.4% at a urea concentration of 0.05% and +18.3% at a urea concentration of 0.1%. Rates of deviation from the true value converged within ±10% were observed as follows: +7.4% at a urea concentration of 0.5%, +7.4% at a urea concentration of 1%, -1.0% at a urea concentration of 5%, and -8.5% at a urea concentration of 10%. A negative deviation of 15% or more was observed when the urea concentration was 15% or more. Based on these results, it was understood that the measured value approaches the true value through the addition of an appropriate amount of urea.

In Fig. 1, the horizontal axis shows added urea concentration in the diluting solution (%), while the vertical axis shows the observed value of the diacetylspermine concentration (nM). A bar showing 154 nM, the true value of diacetylspermine concentration in the control urine sample, is also provided. According to Fig. 1, an added urea concentration of approximately 0.5% to approximately 10% can be considered effective.

The concentration range for urea that was effective in giving accurate diacetylspermine concentration was investigated in more detail. Urea was added to a solution containing 0.3% BSA, 0.9% sodium chloride and 0.2 M MES (pH 6.2) to give solutions having the following urea concentrations: 0.1%, 0.2%, 0.3%, 0.4%, 11%, 12%, 13%, 14%, and 15%. Each urea solution thus obtained was used as a diluting solution. Using diluting solutions with various urea concentrations, standard solutions were prepared containing diacetylspermine at the following concentrations: 0 nM, 10 nM, 50 nM, 200 nM, 400 nM, and 1000 nM Control urine samples containing 120 nM and 238 nM diacetylspermine as determined with the above-mentioned Reagent No. 1 (Reference Example 4) and Reagent No. 2 (Reference Example 5) were used to evaluate the effect of urea. The control urine samples consist of pooled urine from healthy volunteers, and the diacetylspermine concentration of which was evaluated and verified through the use of a calibration curve created using a standard preparation quantified by mass spectrometry. For each of the above-mentioned urea concentrations, 6 standard solutions and urine samples were measured of absorbance differences measured by adding thereto Reagent No. 1 and Reagent No. 2 in a Hitachi model 7070 automatic testing unit, and the diacetylspermine concentration in each urine sample was calculated using the calibration curve obtained for each urea concentration. The optimum concentration of added urea was determined using the rate of deviation between these measured values and true values. These results are shown in Table 2.

**Table 2**

| Urea % | Measured Value nM | Deviation % |
|---|---|---|
| 0.1 | 138.2 | +15.1 |
| 0.2 | 135.5 | +12.9 |
| 0.3 | 133.4 | +11.2 |
| 0.4 | 130.9 | +9.1 |
| Standard Value | 120 | |
| 11 | 214.8 | -9.8 |
| 12 | 206.5 | -13.8 |
| 13 | 194.3 | -18.4 |
| 14 | 192.0 | -19.3 |
| 15 | 187.9 | -21.1 |
| Standard Value | 238 | |

According to Table 2, at a urea concentration of 0.1%, the observed concentration of the control urine sample containing 120 nM diacetylspermine was 138.2 nM, and the rate of deviation from the true value was +15.1%. In contrast, the rate of deviation from the true value was +12.9 % at a urea concentration of 0.2%, +11.2% at a urea concentration of 0.3%, and +9.1% at a urea concentration of 0.4%, which were within the range of ±15% where the addition of urea was effective. At a urea concentration of 15%, the observed concentration of the control urine sample containing 238 nM diacetylspermine was 187.9 nM, and the rate of deviation from the true value was -21.1%, while the rates of deviation from the true value were -19.3% and -18.4% for urea concentrations of 14% and 13%, respectively. The observed concentration of the control urine sample containing 238 nM diacetylspermine was 206.5 nM for a urea concentration of 12%, the rate of deviation from the true value was -13.8%, and the rate of deviation converged within ±15%.

Summarizing the above results, the effective range of urea that gives observed value whose the rate of deviation is within ±15% of the true valeue is considered to be 0.2 to 12%.

### 1-2. Investigation concerning Standard Solutions and Diluting Solutions containing BSA

Urea content was settled at 5% based on the results of 1-1 above. Accordingly, BSA was added to solutions containing 5% urea, 0.9% sodium chloride and 0.2 M MES (pH 6.2) to give solutions having the following BSA concentrations: 0%, 0.01 %, 0.1%, 0.3%, 1%, and 3%. Each BSA solution thus obtained was used as a diluting solution. Using diluting solutions with various BSA concentrations, standard solutions containing diacetylspermine in the following concentrations were prepared: 0 nM, 10 nM, 50 nM, 200 nM, 400 nM, and 1000 nM. A control urine sample containing 154 nM diacetylspermine as determined with the above-mentioned Reagent No. 1 (Reference Example 4) and Reagent No. 2 (Reference Example 5) was used to evaluate the effect of BSA. The control urine sample consist of pooled urine from healthy volunteers, and the diacetylspermine concentration of which was evaluated and verified through the use of a calibration curve created using a standard preparation quantified by mass spectrometry. For each of the above-mentioned BSA concentrations, 6 standard solutions and urine samples were measured of absorbance differences by adding thereto Reagent No. 1 and Reagent No. 2 in a Hitachi model 7070 automatic testing unit, and the diacetylspermine concentration in the urine sample was calculated using the calibration curve obtained for each BSA concentration. The effect of the concentration of BSA added was examined using the ratio of the measured values to the true value (concordance rate). These results are shown in Table 3.

**Table 3**

| BSA | Measured Value nM | Concordance Rate % |
|---|---|---|
| 0% | 142.2 | 99.2 |
| 0.01% | 139.5 | 97.3 |
| 0.1% | 142.3 | 99.3 |
| 0.3% | 143.3 | 100.0 |
| 1% | 137.5 | 96.0 |
| 3% | 126.8 | 88.5 |
| Standard Value | 154 | |

As can be ascertained from Table 3 above, values of nearly 100% were obtained for every BSA concentration, except for the slight decrease in the observed value when BSA concentration was 3%.

### [EXAMPLE 2]

### <Measurement of Urinary Diacetylspermine>

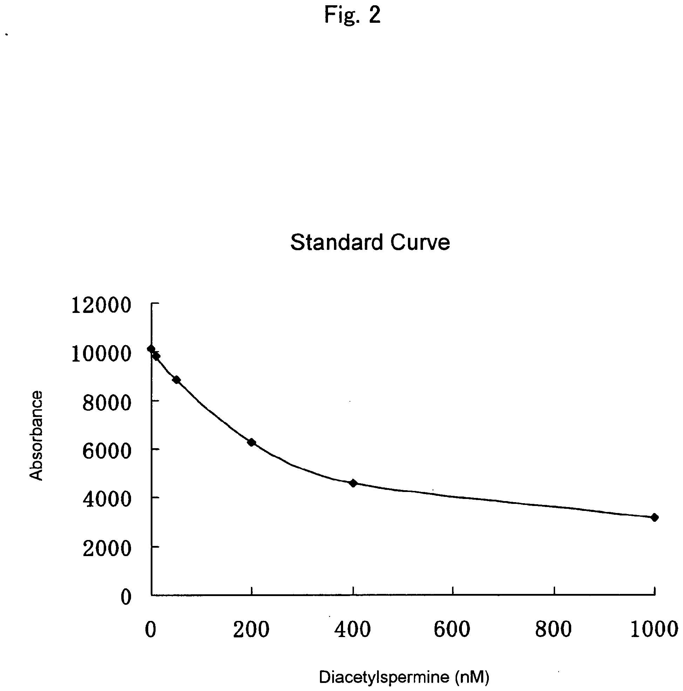

In the present example, 0.2 M MES (pH 6.2) containing 5% urea, 0.3% BSA and 0.9% sodium chloride was used as a diluting solution. Using this diluting solution, standard solutions were prepared that contain diacetylspermine in the following concentrations: 0 nM, 10 nM, 50 nM, 200 nM, 400 nM, and 1000 nM. A calibration curve was created from the standard solutions corresponding to these 6 concentrations (points). The results are shown in Fig. 2. The horizontal axis of Fig. 2 shows diacetylspermine concentration (nM), while the vertical axis shows absorbance (OD×10000). As is evident from Fig. 2, a reduction in absorbance depending on urinary diacetylspermine concentration was observed.

In the next step, we used two control urine samples containing 154nM and 240nM diacetylspermine, respectively. These control urine samples were pooled urine samples obtained from two groups of healthy volunteers, and the diacetylspermine concentration of which was measured with the above-mentioned Reagent No. 1 (Reference Example 4) and Reagent No. 2 (Reference Example 5). The concentrations of diacetylspermine of these samples were evaluated and verified through the use of a calibration curve created using a standard preparation quantified by mass spectrometry. The diacetylspermine concentrations in urine sample A (154nM) and urine sample B (240nM) were measured (n=4) using the above-mentioned standard solution as the reference. Results are shown in Table 4.

**Table 4**

| N=4 | Sample A (154 nM) | Sample B (240 nM) |
|---|---|---|
| 1 | 154.1 | 259.2 |
| 2 | 152.8 | 245.9 |
| 3 | 154.9 | 256.0 |
| 4 | 148.7 | 255.4 |
| Average | 152.6 | 254.1 |

As is evident in Table 4 above, when 0.2 M MES (pH 6.2) containing 5% urea, 0.3% BSA and 0.9% sodium chloride was used as a standard solution or diluting solution, there was only a slight margin of error between the obtained measured values and the actual diacetylspermine concentration. By using the above-mentioned solution as a standard solution or diluting solution in this way, the effect of urine sample matrix can be ignored, and the deviation of measured values from true values that occurs when normal saline solution is used can be avoided.

### [EXAMPLE 3]

### <Investigation into Components in the Fluid for Preparing Standard Solution >

In the present example, standard solutions were prepared using those substances which could be effective in providing accurate measurement values in diacetylspermine measurement, and the prepared standard solutions were used to measure the diacetylspermine concentration of urine samples.

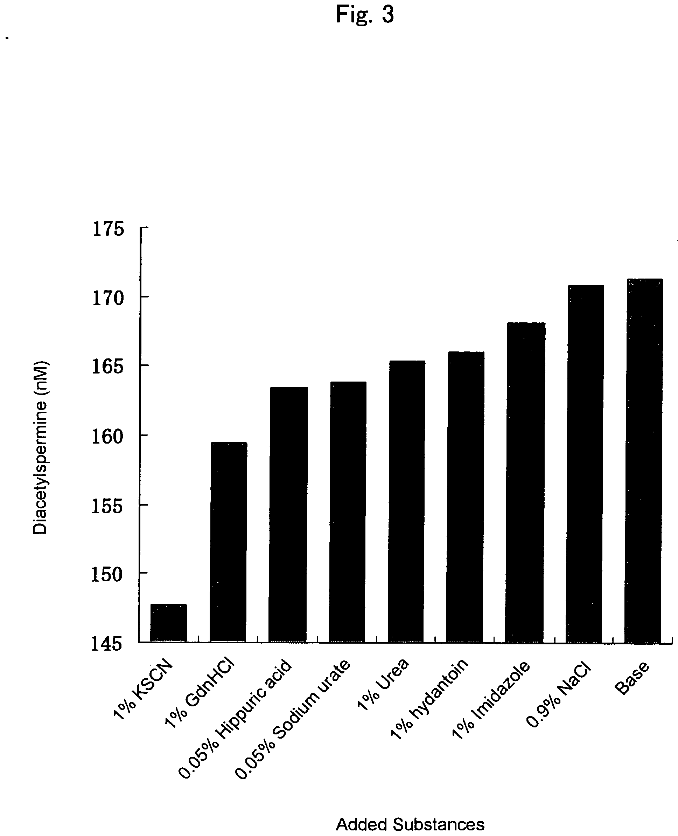

First, each of the test substances was individually added to a solution containing 0.3% BSA, 0.9% sodium chloride and 0.2 M MES (pH6.2) to give the following concentrations to thereby obtain diluting solutions: 1% potassium thiocyanate (KSCN), 1% guanidinium hydrochloride (GdnHCl), 1% imidazole, 1% hydantoin, and 0.05% hippuric acid or 0.05% sodium urate (uric acid-Na). As a positive control, a solution containing 1% urea was used. As negative control, a solution (base) containing no substances other than BSA, sodium chloride and MES, and a normal saline solution (0.9% NaCl) were used. Using these diluting solutions, standard solutions were prepared which contained diacetylspermine at the following concentrations: 0 nM, 10 nM, 50 nM, 200 nM, 400 nM, and 1000 nM A control urine sample containing 154 nM diacetylspermine as determined with the above-mentioned Reagent No. 1 (Reference Example 4) and Reagent No. 2 (Reference Example 5) was used to evaluate the effect of the above-mentioned substances on measured values. The control urine sample consists of pooled urine from healthy volunteers, and the diacetylspermine concentration of which was evaluated and verified through the use of a calibration curve created using a standard preparation quantified by mass spectrometry. For each of the above-mentioned substances, 6 standard solutions and urine samples were measured of absorbance differences by adding Reagent No. 1 and Reagent No. 2 in a Hitachi model 7070 automatic testing unit, and the diacetylspermine concentration in each urine sample was calculated using the calibration curve obtained for each substance. These results are shown in Table 5 and Fig. 3 (Fig. 3 is a graph showing the results of Table 5 wherein the horizontal axis shows the added substance and the vertical axis shows the measured value (nM) of diacetylspermine concentration).

**Table 5**

| | | Measured Value nM | Deviation % |
|---|---|---|---|
| 1% | KSCN | 147.7 | -4.1 |
| 1% | GdnHCl | 159.4 | +3.5 |
| 0.05% | Hippuric Acid | 163.4 | +6.1 |
| 0.05% | Sodium Urate | 163.8 | +6.3 |
| 1% | Urea | 165.4 | +7.4 |
| 1% | Hydantoin | 166.0 | +7.8 |
| 1% | Imidazole | 168.1 | +9.1 |
| 0.9% | NaCl | 170.9 | +11.0 |
| | Base | 171.3 | +11.3 |
| Standard Value | | 154 | |

As shown in Table 5 and Fig. 3, when a base (a negative control) was used, the observed concentration in the control urine sample containing 154 nM diacetylspermine was 171.3 nM, and the rate of deviation from the true value was +11.3%, exceeding 10%. Use of a normal saline solution (0.9% NaCl) also yielded a value of 170.9 nM, which was higher than the true value, and deviation exceeding 10% was observed. By contrast, addition of 1% imidazole, 1% hydantoin, 0.05% sodium urate, 0.05% hippuric acid, 1% guanidinium hydrochloride, or 1% potassium thiocyanate yielded respective observed values of 168.1 nM, 166.0 nM, 163.8 nM, 163.4 nM, 159.4 nM, and 147.7 nM. These observed values were close to 165.4 nM (which is the result obtained when 1% urea is added), and the rate of deviation from the true values was within ±10%.

### [EXAMPLE 4]

### <Addition of Urea to Reagent No. 1>

The effect of adding urea to Reagent No. 1 was investigated.

Specifically, urea was added to Reagent No. 1 (Reference Example 4) to give a concentration of 0.2% or 1%. Concentrations of diacetylspermine in four samples were measured with the above-mentioned Reagent No. 1 (Reference Example 4) and Reagent No. 2 (Reference Example 5), and their diacetylspermine concentrations were evaluated and verified through the use of a standard curve created using a standard preparation quantified by mass spectrometry. The diacetylspermine concentrations of these specimens were as follows: 475.3 nM (Specimen A), 477.1 nM (Specimen B), 612.2 nM (Specimen C), and 577.5 nM (Specimen D). These urine samples were diluted 5-fold with purified water.

0.2 M MES (pH 6.2) containing 5% urea, 0.3% BSA, and 0.9% sodium chloride was used as a diluting solution to prepare standard solutions containing diacetylspermine in the following concentrations: 0 nM, 10 nM, 50 nM, 200 nM, 400 nM, and 1000 nM. A calibration curve was produced from the standard solutions corresponding to these 6 concentrations (points).

The diacetylspermine concentrations in the diluted urine samples were measured using urea-added Reagent No. 1. The results are shown in Table 6.

**Table 6**

| | Urea 0.2% | | | Urea 1.0% | | |
|---|---|---|---|---|---|---|
| Specimen No. | True Value nM | Purified Water * 5-fold Dilution nM | Concordance % | True Value nM | Purified Water * 5-fold Dilution nM | Concordance % |
| A | 475.3 | 428.3 | 90.1 | 475.3 | 462.2 | 97.2 |
| B | 477.1 | 431.5 | 90.5 | 477.1 | 454.6 | 95.3 |
| C | 612.2 | 565.0 | 92.3 | 612.2 | 581.1 | 94.9 |
| D | 577.5 | 547.7 | 94.8 | 577.5 | 551.6 | 95.5 |

As Table 6 shows, when 0.2% urea- or 1% urea-added Reagent No. 1 is used to measure urine samples diluted 5-fold with purified water, a concordance of 90% or more with respect to true values was observed at both 0.2 % and 1% urea. These results revealed that accurate measurement values can be obtained even when urea is added to the reagent itself.

### INDUSTRIAL APPLICABILITY

According to the present invention, in immunologically measurement of urine components, the quantity of the urine component diacetylspermine can be measured more accurately. When positive or negative diagnosis is made depending on the quantity of a substance which may be used as a diagnosis indicator or early diagnosis marker, the present invention enables more accurate diagnosis. Diacetylspermine is measured by colloidal gold agglutination method which is simple and available at low cost. According to the present invention, the accuracy and ease of use of this method are improved even more.

## Claims

1. Use of a reagent in immunological measurement of a urine component, wherein said reagent comprises at least one substance selected from urea, potassium thiocyanate, guanidinium hydrochloride, imidazole, hydantoin, hippuric acid and sodium urate, said urine component is diacetylspermine and said immunological measurement is carried out by a colloidal gold agglutination method.

2. The use according to claim 1, wherein the protein concentration of said reagent is 0.01 to 1 w/v %.

3. The use according to claim 2, wherein said protein is bovine serum albumin.

4. The use according to any one of claims 1 to 3, wherein the salt concentration of said reagent is 0.01 to 10 w/v %.

5. The use according to any one of claims 1 to 4, wherein said reagent is a standard solution, diluting solution, or reaction reagent.

6. The use according to any one of claims 1 to 5, wherein said urine is human urine.

7. A method for immunologically measuring the quantity of a urine component, which comprises the following steps:
(a) adding a reaction reagent to a urine sample or a mixed solution of a urine sample and a diluting solution to generate a reaction solution and then measuring either the change caused by immunological reaction or the reaction product;
(b) adding said reaction reagent to a standard solution or a mixed solution of a standard solution and a diluting solution to generate a reaction solution and then measuring either the change caused by immunological reaction or the reaction product; and
(c) comparing the measurement results of (a) to the measurement results of (b) to determine the quantity of the urine component in the urine sample,
wherein at least one reagent selected from the diluting solution, the standard solution and the reaction reagent comprises at least one substance selected from urea, potassium thiocyanate, guanidinium hydrochloride, imidazole, hydantoin, hippuric acid and sodium urate, and said substance is contained in the reaction solution generated in (b) and wherein said urine component is diacetylspermine and said immunological measurement is carried out by a colloidal gold agglutination method.

8. The method according to claim 7, wherein the protein concentration of the reaction solution generated in (b) is 0.00025 to 0.03 w/v %.

9. The method according to claim 8, wherein said protein is bovine serum albumin.

10. The method according to any one of claims 7 to 9, wherein the salt concentration of the reaction solution generated in (b) is 0.00025 to 0.3 w/v %.

11. The method according to any one of claims 7 to 10, wherein said urine is human urine.

12. The method according to any one of claims 7 to 11, wherein at least one reagent selected from the diluting solution, the standard solution and the reaction reagent comprises at least urea, and said urea is contained in the reaction solution generated in (b).

## Patentansprüche

1. Verwendung eines Reagens in immunologischer Messung von einer Urinkomponente, wobei genanntes Reagens mindestens eine Substanz, ausgewählt aus Harnstoff, Kaliumthiocyanat, Guanidiniumhydrochlorid, Imidazol, Hydantoin, Hippursäure und Natriumurat umfasst, genannte Urinkomponente Diacetylspermin ist und genannte immunologische Messung durch ein kolloidales Goldagglutinationsverfahren durchgeführt wird.

2. Verwendung gemäß Anspruch 1, wobei die Proteinkonzentration von genanntem Reagens 0,01 bis 1 G/V % ist.

3. Verwendung gemäß Anspruch 2, wobei genanntes Protein Rinderserumalbumin ist.

4. Verwendung gemäß irgendeinem der Ansprüche 1 bis 3, wobei die Salzkonzentration von genanntem Reagens 0,01 bis 10 G/V % ist.

5. Verwendung gemäß irgendeinem der Ansprüche 1 bis 4, wobei genanntes Reagens eine Standardlösung, Verdünnungslösung oder ein Reaktionsreagens ist.

6. Verwendung gemäß irgendeinem der Ansprüche 1 bis 5, wobei genannter Urin humaner Urin ist.

7. Verfahren zur immunologischen Messung der Quantität einer Urinkomponente, welches die folgenden Schritte umfasst:
(a) Zufügen eines Reaktionsreagens zu einer Urinprobe oder einer gemischten Lösung aus einer Urinprobe und einer Verdünnungslösung, um eine Reaktionslösung herzustellen und dann Messen entweder der Veränderung, verursacht durch immunologische Reaktion, oder des Reaktionsprodukts;
(b) Zufügen genannten Reaktionsreagenses zu einer Standardlösung oder einer gemischten Lösung aus einer Standardlösung und einer Verdünnungslösung, um eine Reaktionslösung herzustellen und dann Messen entweder der Veränderung, verursacht durch immunologische Reaktion, oder des Reaktionsprodukts; und
(c) Vergleichen der Messergebnisse von (a) mit den Messergebnissen von (b), um die Quantität der Urinkomponente in der Urinprobe zu bestimmen,
wobei mindestens ein Reagens, ausgewählt aus der Verdünnungslösung, der Standardlösung und des Reaktionsreagens, mindestens eine Substanz, ausgewählt aus Harnstoff, Kaliumthiocyanat, Guanidiniumhydrochlorid, Imidazol, Hydantoin, Hippursäure und Natriumurat umfasst, und genannte Substanz in der Reaktionslösung, hergestellt in (b) beinhaltet ist und wobei genannte Urinkomponente Diacetylspermin ist und genannte immunologische Messung durch ein kolloidales Goldagglutinationsverfahren ausgeführt wird.

8. Verfahren gemäß Anspruch 7, wobei die Proteinkonzentration von der Reaktionslösung, hergestellt in (b), 0,00025 bis 0,03 G/V % ist.

9. Verfahren gemäß Anspruch 8, wobei genanntes Protein Rinderserumalbumin ist.

10. Verfahren gemäß irgendeinem der Ansprüche 7 bis 9, wobei die Salzkonzentration von der Reaktionslösung, hergestellt in (b), 0,00025 bis 0,3 G/V % ist.

11. Verfahren gemäß irgendeinem der Ansprüche 7 bis 10, wobei genannter Urin humaner Urin ist.

12. Verfahren gemäß irgendeinem der Ansprüche 7 bis 11, wobei mindestens ein Reagens, ausgewählt aus der Verdünnungslösung, der Standardlösung und dem Reaktionsreagens mindestens Harnstoff umfasst, und genannter Harnstoff in der Reaktionslösung, hergestellt in (b), beinhaltet ist.

## Revendications

1. Utilisation d'un réactif dans un dosage immunologique d'un composant d'urine, dans laquelle ledit réactif comprend au moins une substance choisie parmi les suivantes : urée, thiocyanate de potassium, chlorhydrate de guanidinium, imidazole, hydantoïne, acide hippurique et urate de sodium, ledit composant d'urine est la diacétyl-spermine et ledit dosage immunologique est effectué selon un procédé d'agglutination avec marquage à l'or colloïdal.

2. Utilisation conforme à la revendication 1, dans laquelle la concentration de protéine(s) dans ledit réactif vaut de 0,01 à 1 % p/v.

3. Utilisation conforme à la revendication 2, dans laquelle ladite protéine est de la sérumalbumine bovine.

4. Utilisation conforme à l'une des revendications 1 à 3, dans laquelle la concentration de sel(s) dans ledit réactif vaut de 0,01 à 10 % p/v.

5. Utilisation conforme à l'une des revendications 1 à 4, dans laquelle ledit réactif est une solution étalon, une solution pour dilution ou un réactif de réaction.

6. Utilisation conforme à l'une des revendications 1 à 5, dans laquelle ladite urine est de l'urine humaine.

7. Procédé de mesure immunologique de la quantité d'un composant d'urine, qui comporte les étapes suivantes :
a) ajouter un réactif de réaction à un échantillon d'urine, ou à une solution qui est un mélange d'un échantillon d'urine et d'une solution pour dilution, pour en faire une solution de réaction, et mesurer ensuite soit le changement provoqué par la réaction immunologique, soit la quantité de produit de réaction ;
b) ajouter ledit réactif de réaction à une solution étalon, ou à une solution qui est un mélange d'une solution étalon et d'une solution pour dilution, pour en faire une solution de réaction, et mesurer ensuite soit le changement provoqué par la réaction immunologique, soit la quantité de produit de réaction ;
c) et comparer les résultats de mesure obtenus dans l'étape (a) avec les résultats de mesure obtenus dans l'étape (b) pour déterminer la quantité du composant d'urine dans l'échantillon d'urine ;
étant entendu qu'au moins un réactif choisi parmi la solution pour dilution, la solution étalon et le réactif de réaction comprend au moins une substance choisie parmi les suivantes : urée, thiocyanate de potassium, chlorhydrate de guanidinium, imidazole, hydantoïne, acide hippurique et urate de sodium, et que cette substance est contenue dans la solution de réaction formée dans l'étape (b), que ledit composant d'urine est la diacétyl-spermine, et que ledit dosage immunologique est effectué selon un procédé d'agglutination avec marquage à l'or colloïdal.

8. Procédé conforme à la revendication 7, dans lequel la concentration de protéine(s) dans la solution de réaction formée dans l'étape (b) vaut de 0,00025 à 0,03 % p/v.

9. Procédé conforme à la revendication 8, dans lequel ladite protéine est de la sérumalbumine bovine.

10. Procédé conforme à l'une des revendications 7 à 9, dans lequel la concentration de sel(s) dans la solution de réaction formée dans l'étape (b) vaut de 0,00025 à 0,3 % p/v.

11. Procédé conforme à l'une des revendications 7 à 10, dans lequel ladite urine est de l'urine humaine.

12. Procédé conforme à l'une des revendications 7 à 11, dans lequel au moins un réactif choisi parmi la solution pour dilution, la solution étalon et le réactif de réaction comprend au moins de l'urée, laquelle urée est contenue dans la solution de réaction formée dans l'étape (b).
